# EUROPÄISCHE PATENTANMELDUNG

(11) **EP 3 961 215 A1**
(43) Veröffentlichungstag der Anmeldung: **02.03.2022**
(21) Anmeldenummer: 20192550.0
(22) Anmeldetag: 25.08.2020
(51) Int. Cl.: G01N 33/543, G01N 33/569

(54) **VERFAHREN ZUR BESTIMMUNG DER AVIDITÄT VON GEGEN CORONAVIRUS GERICHTETEN ANTIKÖRPERN SOWIE HIERZU GEEIGNETE TESTKITS**

(71) Anmelder: Mikrogen GmbH, 82061 Neuried (DE)
(72) Erfinder: BAUER, Georg, 79104 Freiburg (DE); SOUTSCHEK, Erwin, 82335 Berg/Farchach (DE); MOTZ, Manfred, 80689 München (DE)
(74) Vertreter: Lederer & Keller Patentanwälte Partnerschaft mbB

(57) **Zusammenfassung**

Offenbart wird ein Verfahren zur Bestimmung der Avidität von gegen ein Coronavirus gerichteten Antikörpern, das folgende Schritte umfasst:
a) Bereitstellung von mindestens zwei festen Phasen, wobei auf jeder festen Phase in gleicher Weise und in gleicher Menge wenigstens zwei verschiedene Antigene von SARS - CoV-2 immobilisiert sind, und auf jeder festen Phase die verschiedenen Antigene räumlich voneinander getrennt sind,
b) Inkubation der festen Phasen aus a) mit einer Antikörper enthaltenden Lösung,
c) Inkubation einer der festen Phasen in einer Lösung, die nicht die Bindung von Antikörpern mit niedriger Avidität an die Antigene wesentlich reduziert, sowie
d) Inkubation mindestens einer weiteren festen Phase in mindestens einer Lösung, wobei diese Lösung Harnstoff in einer Konzentration aufweist, die die Bindung von Antikörpern mit niedriger Avidität an die Antigene reduziert, und
e) Detektion der an die festen Phasen gebundenen Antikörper,
worin die Konzentration an Harnstoff so eingestellt ist, dass nur ein Teil der gebundenen Antikörper abgelöst wird.

## Beschreibung

Die durch ein spezielles, aggressives Coronavirus verursachte COVID-19-Pandemie hat der Weltwirtschaft schwersten Schaden zugefügt. Ein wesentlicher Aspekt für geeignete Gegenmaßnahmen ist eine gute und zuverlässige Diagnostik, bei der festgestellt werden kann, ob eine Infektion mit SARS-CoV-2 vorliegt. Für die akute Diagnostik wird üblicherweise auf die Polymerasekettenreaktion zurückgegriffen, weil dort die Nukleinsäure nachgewiesen werden kann. Die Polymerasekettenreaktion ist in den Frühstadien einer Infektion das Mittel der Wahl.

Ein anderer Aspekt ist die Frage, ob schon eine Infektion mit SARS-CoV-2 vorgelegen hat und ob möglicherweise eine Immunität gegenüber dem gefährlichen Virus vorliegt.

Humane Coronaviren wurden bereits in den 60er Jahren des letzten Jahrhunderts bei Erkältungskrankheiten entdeckt. Elektronenmikroskopische Aufnahmen zeigen Viruspartikel, die von einer Membranhülle mit eingelagerten Proteinen umgeben waren, wodurch sie wie von einem "Strahlenkranz" (lateinisch: corona) umgeben erschienen. Die Membran-umhüllten Virionen der Coronaviren haben ein einzelsträngiges RNA-Genom in +-Strang-Orientierung, das mit N'-Proteinen als Nukleocapsid im Inneren der Partikel vorliegt. Das Nukleocapsid ist von einer Membranhülle umgeben, in welche die Glykoproteine S, HE und M eingelagert sind. Die klassischen Coronaviren waren lange bekannt, spielten aber bei Erkältungserkrankungen eher eine untergeordnete Rolle, da schwere Krankheitsverläufe nicht bekannt wurden.

Ende 2019, Anfang 2020 entwickelte sich in China eine Coronavirusmutante, die schwere Krankheitsverläufe hervorrufen kann, weil insbesondere bei Patienten mit Vorerkrankungen schwere Lungenschäden auftreten können und eine gesteigerte Letalität beobachtet wird.

Die Diagnostik von SARS-CoV-2 wird hauptsächlich durch zwei Aspekte erschwert. Einerseits sind Kreuzreaktivitäten mit den bereits vorbekannten, relativ ungefährlichen Coronaviren zu beobachten und andererseits wurde festgestellt, dass nach durchlaufener Infektion mit SARS-CoV-2 nicht regelmäßig die sonst übliche Immunität beobachtet werden kann.

### Hintergrund

Die klassische serologische Diagnostik einer Infektion beruht auf der spezifischen Bestimmung von Antikörpern der Klassen IgM und IgG, die gegen Erreger-spezifische Struktur- oder Regulationsproteine gerichtet sind. Bei der Entwicklung einer immunologischen Antwort treten zunächst B-Zellen auf, die gegen spezifische Epitope des Erregers gerichtete IgM-Antikörper an ihrer Oberfläche tragen. Diese B-Zellen teilen sich aufgrund spezifischer Stimulation durch das passende Antigen weiter, wobei ein Teil dieser Zellpopulation zu Plasmazellen differenziert. Diese produzieren dann die entsprechenden **IgM**-Antikörper in hoher Konzentration. Eine erfolgreiche Immunantwort wird weiterhin dadurch ermöglicht, dass sich einige der **IgM**-tragenden B-Zellen durch genomische Umlagerungen (Immunglobulin-Klassenswitch) zu **IgG**-tragende B-Zellen weiterentwickeln. Diese B-Zell-Population bildet ebenfalls Plasmazellen aus, die für die Produktion von IgG
verantwortlich sind. Um eine langanhaltende spezifische Immunantwort zu gewährleisten, entwickeln sich schließlich Gedächtniszellen, die es ermöglichen, zu einem späteren Zeitpunkt bei Bedarf die Immunabwehr zu reaktivieren. Da zwischen der ersten Ausbildung von IgG-bildenden B-Zellen und dem Etablieren von Gedächtniszellen in der Regel klonale Selektion zur Steigerung der Affinität der IgG-Moleküle stattfindet ("Affinitätsreifung"), kann eine auf der Reaktivierung von Gedächtniszellen basierende Immunantwort wesentlich effizienter agieren als die initiale Immunantwort.

Die klonale Selektion stellt einen komplexen, langandauernden Prozess dar, der einer optimalen, und gleichzeitig langanhaltenden Antigenkonzentration bedarf. Bei diesem Prozess entstehen aufgrund von Zufallsmutationen B-Zellklone mit variierender Affinität ihres jeweiligen IgGs für die Zielepitope. Klone mit IgG höherer Affinität haben besseren Zugriff auf das verfügbare Antigen. Sie überleben deshalb und teilen sich weiter, während Klone mit niedrigerer Affinität des IgG aufgrund des durch Kompetition bedingten Antigen-Mangels apoptotischen Zelltod erleiden. Durch mehrere Zyklen dieses Selektionsprozesses überleben schließlich B-Zellklone, die IgG von sehr hoher Affinität generieren können. Diese Klone bilden entweder Plasmazellen für umfassende IgG-Bildung oder bleiben als Gedächtniszellen erhalten. Dies ist der Zustand einer ausgereiften Immunantwort und damit die Basis einer lang anhaltenden und protektiven Immunität.

Falls klonale Selektion und damit einhergehende Affinitätsreifung nicht stattfinden oder einen Abbruch erleiden (z. B. durch Limitierung des Antigens), kann daraus ein Abbruch der serologischen Antwort, ein Ausbleiben der Bildung von Gedächtniszellen, oder langfristig auch ein Verlust der initial erworbenen Immunität resultieren.

Der Mechanismus der Etablierung der Immunantwort führt anfangs zu einer strikten Abfolge des Auftretens von IgM-tragenden B-Zellen gefolgt von IgG-tragenden B-Zellen. Nachfolgend verschwinden IgM-tragenden B-Zellen, während eine Population IgGtragender B-Zellen erhalten bleibt. Diese strikte Reihenfolge von IgM und IgG bei den initialen Prozessen auf der Ebene von B-Zellen wurde in der Vergangenheit häufig auch auf das Muster des Auftretens von IgM und IgG im Serum nach Infektion mit einem Erreger übertragen und als zentrale Grundlage der Differenzierung zwischen akuten, kürzlich erfolgten und länger zurückliegenden Infektionen angewandt. Der Berechtigung dieser Anwendungsweise steht jedoch die Realität, der diagnostischen Praxis entgegen. Dem diagnostischen Praktiker ist die hohe Variabilität der serologischen Antworten nach akuter Infektion seit langem bekannt. Diese Variabilität beruht darauf, dass nach dem Klassenswitch der Immunglobuline eine Vielzahl von Prozessen nebeneinander ablaufen, die zu unterschiedlichen Ergebnissen beim Auftreten der Immunglobuline im Serum führen können, ohne dass dabei die Reihenfolge IgM zu IgG beim ursprünglichen Klassenswitch in Frage gestellt würde.

Ein Paradebeispiel zur Demonstration der serologischen Variabilität ist die EBV-Serologie. So wurde zu Beginn der 90iger Jahre erkannt, dass a) in der Regel bei akuten Infektionen IgG und IgM gleichzeitig im Serum nachweisbar werden; b) in einer bestimmten Anzahl von Fällen IgM niemals detektierbar ist (dafür gibt es mehrere Gründe), c) in einer bestimmten Zahl von Fällen die IgM-Antwort sogar erst nach der IgG-Antwort nachweisbar sein kann und d) in seltenen Fällen IgM-Antworten bis zu einem Jahr oder sogar länger persistieren können. Diese Variabilität der serologischen Antwort wurde inzwischen auch für eine Vielzahl anderer Erreger nachgewiesen.

Die Problematik variabler IgM- und IgG-Antworten konnte durch das Einbeziehen der Aviditätsbestimmung weitgehend gelöst werden. Da die Affinitätsreifung einen regelmäßig auftretenden und unidirektionalen Vorgang darstellt, kann durch die Bestimmung der Affinität eine klare Unterscheidung zwischen akuten, intermediären und lang zurückliegenden Infektionen erzielt werden. Allerdings stellt die exakte Bestimmung der Affinität, bei der sowohl die Hin- als auch die Rückreaktion des IgG zum Antigen berücksichtigt werden muss, ein biophysikalisches Messproblem dar, das für den Einsatz in der Routinediagnostik ungeeignet ist. Dieses Dilemma wurde durch die Beschränkung auf die Bestimmung der Rückreaktion gelöst. Es wurde also im Wesentlichen die Bindungsfestigkeit zwischen IgG und Epitopen bestimmt. Für diesen Prozess wird seitdem in der Praxis der Begriff **"Aviditätsbestimmung"** (lat. aviditas = Gier) verwendet.

Zur Bestimmung der Avidität ist ein streng quantitatives Testsystem notwendig. Der IgG-Bindungstest wird dabei parallel in wenigstens zwei identischen Ansätzen gestartet. Nach der ersten Inkubation der Serumverdünnung mit dem Antigen-Träger erfolgt im Kontrollansatz der übliche Waschschritt, im zweiten Ansatz werden durch eine definierte Konzentration an Harnstoff (in der Regel im Bereich von 6-7 M) niedrigavide Antikörper abgelöst, während hochavide Antikörper am Antigen binden bleiben. In beiden Reaktionsansätzen erfolgt dann die Bestimmung der gebundenen Antikörper durch die bekannten geeigneten Methoden. Das Verhältnis des Reaktionswertes des Ansatzes mit Harnstoffbehandlung zum Wert des Kontrollansatzes wird üblicherweise als **Aviditätsindex** bezeichnet. Ein niedriger Aviditätsindex spricht für eine akute Infektion, ein hoher Index für eine länger zurückliegende Infektion. Dieses Verfahrens kann in entsprechenden Automaten durchgeführt und präzis quantifiziert werden.

Gegenstand der vorliegenden Erfindung ist daher ein Verfahren zur Bestimmung der Avidität von gegen ein Coronavirus gerichteten Antikörpern, das folgende Schritte umfasst:
a) Bereitstellung von mindestens zwei festen Phasen, wobei auf jeder festen Phase in gleicher Weise und in gleicher Menge wenigstens zwei verschiedene Antigene von SARS - CoV-2 immobilisiert sind, und auf jeder festen Phase die verschiedenen Antigene räumlich voneinander getrennt sind,
b) Inkubation der festen Phasen aus a) mit einer Antikörper enthaltenden Lösung,
c) Inkubation einer der festen Phasen in einer Lösung, die nicht die Bindung von Antikörpern mit niedriger Avidität an die Antigene wesentlich reduziert, sowie
d) Inkubation mindestens einer weiteren festen Phase in mindestens einer Lösung, wobei diese Lösung Harnstoff in einer Konzentration aufweist, die die Bindung von Antikörpern mit niedriger Avidität an die Antigene reduziert, und
e) Detektion der an die festen Phasen gebundenen Antikörper,
wobei die Konzentration an Harnstoff so eingestellt ist, dass nur ein Teil der gebundenen Antikörper abgelöst wird.

Ein wesentlicher Aspekt der vorliegenden Erfindung beruht also darauf, dass bestimmte Antigene in im Wesentlichen identischer Anordnung und Menge auf mindestens 2, vorzugsweise mehrere festen Phasen aufgebracht werden. Diese Antigene werden dann mit der Lösung in Kontakt gebracht, die die nachzuweisenden Antikörper enthält. Gegebenenfalls folgt hierauf ein Waschschritt. Anschließend wird eine feste Phase in einer Lösung inkubiert, die auf das Bindungsverhalten von Antigen-Antikörper keinen Einfluss hat (Verfahrensschrift c)), und eine andere feste Phase wird mit einer Lösung inkubiert, die einen Wirkstoff enthält, der die Bindung zwischen Antigen und Antikörper schwächt. Hierzu wird erfindungsgemäß eine Harnstofflösung eingesetzt, wobei in bevorzugter Ausführungsform mehrere unterschiedliche Versuchsansätze durchgeführt werden, bei denen sich die jeweils eingesetzten Harnstoffkonzentrationen in den Bereich zwischen 3 molar und bis zu 7 molar bewegen. Diese Harnstoffkonzentrationen betragen in besonders bevorzugter Ausführungsform 3,1 molar, 3,8 molar, 4,5 molar, 5,0 molar, 5,5 molar, 6,1 molar, 6,8 molar und 6,9 molar.

Die Antigene werden auf feste Phasen aufgebracht, die so ausgestaltet sind, dass sie gut eine Bindung zwischen dem Trägermaterial und dem Antigen ermöglichen. Bevorzugt handelt es sich hierbei um Nitrocellulosestreifen, Nylonstreifen oder Polyvinylidenfluorid. Die Antigene können aber auch an übliche Mikrotiterplatten gebunden werden oder an Polystyrolkugeln, wenn die Reaktionen in üblichen ELISA-Formaten durchgeführt werden. In bestimmten Ausführungsformen können die festen Phasen auch Kügelchen darstellen, die magnetisierbar sind, so dass eine leichte Abtrennung der Kügelchen von dem Reaktionsmedium erzielt werden kann.

Die bei dem Verfahren eingesetzten festen Phasen enthalten darauf immobilisiert solche Antigene, die für die Diagnostik von SARS-CoV-2 besonders geeignet sind. Hierbei handelt es sich in bevorzugter Ausführungsform um ein SARS-CoV-2-Nukleoprotein, eine SARS-CoV-2-Rezeptor-bindende Domäne des S1-Proteins und/oder ein SARS-CoV-2-Oberflächenprotein 1. Selbstverständlich müssen nicht unbedingt die vollständigen Proteine eingesetzt werden. Es können auch Peptidsequenzen ausgewählt werden, die die relevanten Antigenbereiche beinhalten.

In einer weiteren bevorzugten Ausführungsform beinhalten die festen Phasen, die bei den erfindungsgemäßen Verfahren eingesetzt werden, auch Antigen von saisonalen Coronaviren. In bevorzugter Ausführungsform sind diese ausgewählt aus den Antigenen 229I, NL63, OC43 und HKU1 oder den entsprechenden relevanten Teilen davon, die die maßgeblichen Antigensequenzen enthalten. Diese Antigensequenzen beinhalten die höchstrelevanten Epitope.

Da erfindungsgemäß die einzelnen Phasen mit unterschiedlichen Harnstoffkonzentrationen behandelt werden, muss die Bindung von Antikörpern an Antigenen standardisiert optometrisch bestimmt werden. Dies gelingt mittels handelsüblicher Automaten völlig problemlos. Dadurch können die Verhältnisse der Bindung zwischen Antigen und Antikörper in Abhängigkeit von der jeweiligen Harnstoffkonzentration ohne weiteres ermittelt werden. Aus diesem Verhältnis kann der Aviditätsgrad der jeweiligen Antikörper ohne weiteres ermittelt werden.

In einer besonders bevorzugten Ausführungsform wird ein Antigen von SARS-CoV-2 eingesetzt gegen das neutralisierende Antikörper gebildet werden können. Wenn - beispielsweise bei geimpften Personen- die Avidität der hiergegen gerichteten Antikörper bestimmt wird, kann vorhergesagt werden, ob eine protektive Immunität vorliegt. Voraussetzung hierfür wären hochavide Antikörper gegen solche Antigene des SARS-CoV-2-Virus, die das Virus bei Infektion neutralisieren und daher die gewünschte Wirksamkeit des Impfstoffs bereitstellen.

In einer weiteren Ausführungsform der vorliegenden Erfindung werden Testkits für die Durchführung des erfindungsgemäßen Verfahrens bereitgestellt. Derartige Testkits enthalten die entsprechenden festen Phasen, vorzugsweise Lineassaystreifen und die zugehörigen Reagenzien, wie Waschpuffer und Puffer mit entsprechenden Mengen an Harnstoff, die eine eindeutige und zuverlässige Bestimmung der Avidität ermöglichen.

Im weiten Sinne betrifft die vorliegende Erfindung die Verwendung von verschiedenen Antigenen, abgeleitet aus SARS-CoV-2, wie auch von anderen, klassischen, saisonalen Coronaviren und die Bestimmung, ob in den Seren von Patienten Antikörper gegen diese Antigene vorhanden sind und welche Avidität die jeweiligen Antikörper haben. Gerade bei SARS-CoV-2 sind verschiedene wissenschaftliche Publikationen erschienen, die eine Anomalie der Antikörperreifung vermuten lassen, wodurch möglicherweise eine nicht hinreichende Immunität erzeugt wird. Durch die Bestimmung der Aviditäten der jeweiligen Antikörper, insbesondere der neutralisierenden Antikörper, kann besser vorhergesagt werden, ob der jeweilige Patient eine entsprechende Immunität hat oder nicht.

Bei der Durchführung der Experimente wurde vorgegangen wie folgt:
SARS-CoV-2 infizierte Personen wurden aufgrund ihrer typischen Krankheitssymptome und durch ein positives PCR für SARS CoV-2 erkannt. Serumproben wurden einmalig oder kinetisch gewonnen und bei -20°C gelagert.
Serumproben wurden über ein kommerziell tätiges Unternehmen erworben.

Serumproben von Blutspendern ohne klinische Symptome, die in der Zeit vor der SARS CoV-2 Pandemie gewonnen worden waren wurden vom Bayrischen Roten Kreuz erworben.

In bevorzugter Ausführungsform wurde mithilfe eines Lineassays mit rekombinanten Proteinen gearbeitet. Die Übertragbarkeit der Erfindung auf andere Verfahren wurde exemplarisch mit einem ELISA mit rekombinanten Proteinen überprüft. Beide Testsysteme wurden von der Anmelderin unter professionellen Bedingungen hergestellt.

Lineassay: Nitrozellulosestreifen tragen in räumlich definierter Anordnung geeignete Konzentrationen an folgenden rekombinant hergestellten Proteinen:
SARS-CoV-2 Nukleoprotein (NP), SARS-CoV-2 Rezeptor-bindende Domäne des S1 Proteins (RBD), SARS-CoV-2 Oberflächenprotein 1 (S1), sowie die Nukleoproteine der saisonalen Coronaviren 229E, NL63, OC43 und HKU1. Bei den saisonalen Coronaviren können die Antigene von einem Stamm abgeleitet sein oder auch von mehreren unterschiedlichen Stämmen. Durch die Technik des Auftragens wurde sichergestellt, dass die Konzentration an Antigen gleichmäßig über den gesamten Bereich der jeweiligen Bande verteilt war. Dies ist eine essenzielle Voraussetzung für eine strikt quantitative Bestimmung der Antikörperkonzentrationen. Auf den Streifen waren außerdem folgende Kontrollsystem aufgebracht: Reaktionskontrolle, Antikörperklassen-Kontrolle und Cut-Off-Kontrolle. Diese Kontrollen sicherten den präzisen Ablauf des Tests und waren die Basis für die Quantifizierung und Bewertung.

Die Kombination von drei für die Diagnostik von SARS CoV-2-Infektionen wichtigen Proteinen mit den Nukleocapsiden von vier saisonalen Coronaviren jeweils auf dem selben Streifen erlaubt das sofortige Erkennen von anamnestischen Reaktionen und hilft, serologische Kreuzreaktionen in einem Gesamtzusammenhang zu bewerten.

Die Lineassays wurden von mithilfe eines Automaten ausgewertet. Dazu wurden die Streifen mit jeweils einer 1/100 Verdünnung von Serum für eine definierte Zeit inkubiert. Danach erfolgten in den Kontrollansätzen mehrere Waschschritte mit Waschpuffer zur Entfernung von Serumresten. Einer der Waschschritte mit Waschpuffer entsprach der Zeit die in den parallelen Ansätzen mit Harnstoff-haltigem Puffer für die Inkubation vorgesehen war. Nach den Waschschritten erfolgte die Zugabe von gamma-Ketten-spezifischem Anti-IgG, an das Peroxidase gekoppelt war. Nach einer definierten Inkubationszeit wurde freier Zweitantikörper weggewaschen und die Konzentration an gebundenem Zweitantikörper über die Enzymreaktion der Peroxidase bestimmt.

Harnstoffbehandlung zur Bestimmung der Bindungsfestigkeit des IgG, das innerhalb der ersten Inkubation an Antigen gebunden hatte erfolgte nach der Inkubation der Streifen mit Seren und einem Waschschritt. Der "Aviditätspuffer" enthielt Harnstoff in den in den Beispielen angegebenen Konzentrationen. Nach drei Minuten Inkubation wurde der Harnstoff-haltige Puffer weggewaschen und die Ansätze analog zu den Kontrollansätzen weiter prozessiert. Der Vergleich der optischen Dichte der Ansätze mit Harnstoffbehandlung mit den jeweiligen Kontrollansätzen ohne Harnstoff erlaubt die Berechnung der Aviditätsindizes. Sie sind ein Maß für die Bindungsfestigkeit des Antikörper-Antigen-Komplexes.

Beim ELISA-Verfahren waren definierte Antigene in den Vertiefungen von Mikrowell-Platten aufgetragen. Der Versuchsablauf erfolgte analog dem oben beschriebenen Verfahren.

### Beispiel 1

### Abbruch der IgG-Bildung und Ausbleiben vollständiger Aviditätsreifung

Für eine erste Analyse der Kinetik der serologischen Antwort und der begleitenden Aviditätsreifung standen Seren aus sechs Verläufen von SARS-CoV-2-Infektionen zur Verfügung, die durch parallelen Nachweis von SARS-CoV-2-RNA mittels PCR gesichert worden waren. Diese Seren wurden zunächst der der klassischen Aviditätsbestimmung mithilfe eines speziell entwickelten Lineassays (Mikrogen GmbH) unterzogen und der jeweilige Verlauf der IgG-Antwort und deren Avidität gemessen.

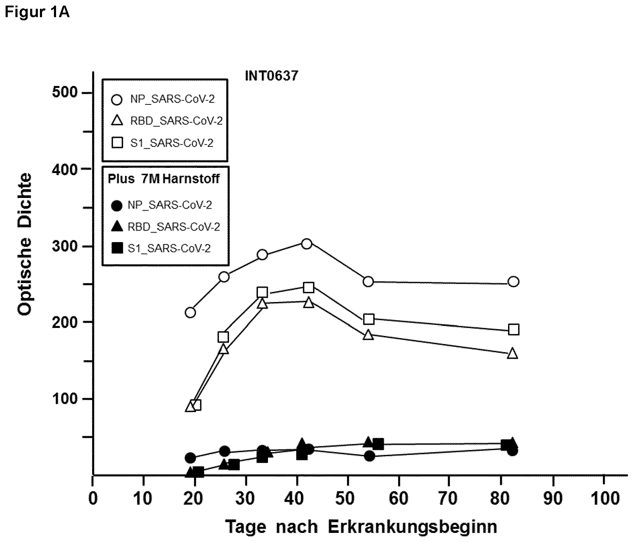

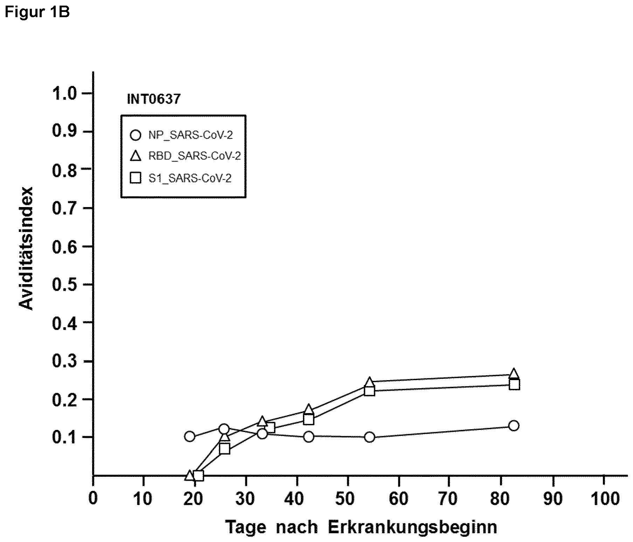

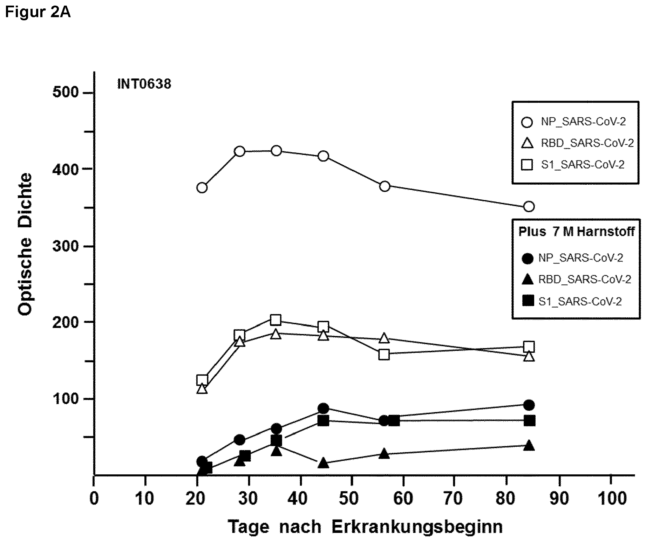

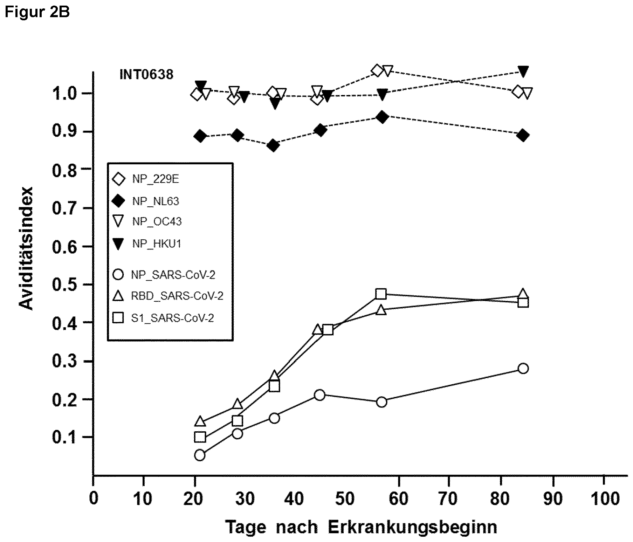

Die zur Verfügung stehenden Fallbeispiele zeigten einen unerwarteten und für Virusinfektionen im Allgemeinen untypischen Verlauf. Dies wird beispielhaft in den Figuren 1-3 dargestellt:
Sehr früh nach Infektion wird ein Plateau der IgG-Antikörperantwort erreicht, wonach ein Abfall der Antikörperkonzentration erfolgt (Figur 1-3). Dieser Abfall ist nur dadurch zu erklären, dass die Produktion neuer IgG-Antikörper ab einem bestimmten Zeitpunkt nachlässt, während der natürliche Abbau im Organismus bereits einsetzt. Die mit Harnstoff behandelten Testansätze weisen in der Regel unerwarteterweise sehr niedrige Werte über den gesamten Verlauf auf. Dies trifft für die Antikörper gegen alle drei Marker zu, i. e. Nukleoprotein (NP), Rezeptor-Bindungs-Domäne (RBD) und Oberflächenprotein S1. Betrachtet man die jeweils ermittelten Aviditätsindizes, so wird besonders leicht erkennbar, dass es sehr bald nach Infektion trotz deutlicher initialer Aviditätsreifung sehr schnell zu einem Nachlassen oder sogar Stillstand der Aviditätsreifung kommt, so dass insgesamt eine weitaus geringere, und damit unvollständigere Aviditätsreifung als bei anderen Virusinfektionen bekannt, vorliegt.

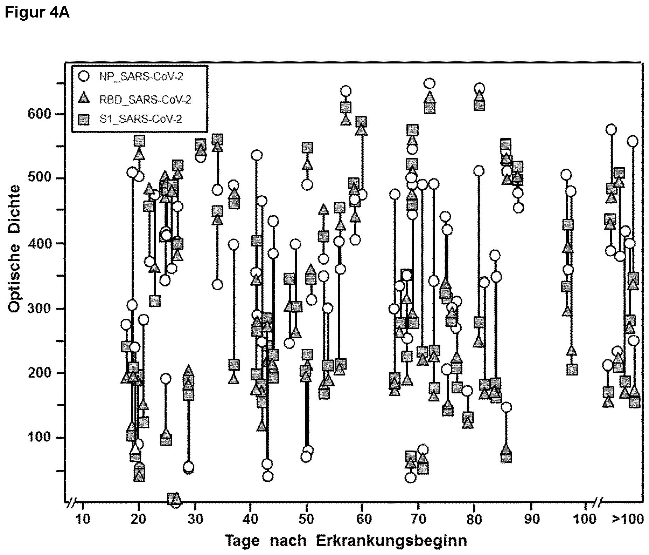

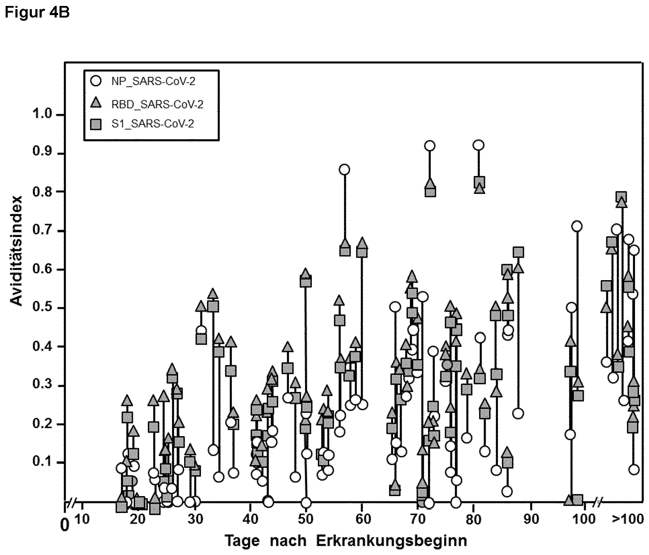

Die Ergänzung der kinetischen Analyse von Verlaufsfällen durch eine große Zahl von Einzelfällen gesicherter SARS-CoV-2-Infektionen (Figur 4) bestätigt diesen Sachverhalt: in der Regel findet sich ein Aviditätsindex in dem üblicherweise für frische Infektionen typischen niedrigen Bereich. Nur wenige Ausnahmefälle erreichen einen für vollständige Aviditätsreifung sprechenden hohen Aviditätsindex. Dennoch ist die grundsätzliche Tendenz zum Anstieg des Aviditätsindizes im Lauf der Zeit nach Erkrankungsbeginn erkennbar.

Abbildung 4 zeigt auch den für die spätere Diskussion wichtigen Befund, dass bei jedem der getesteten Seren Antikörper gegen jedes der drei verwendeten Antigene (NP, RBD, S1) nachweisbar waren, selbst wenn die gemessene optische Dichte des Ansatzes in einem sehr niedrigen Bereich lag.
- *Insgesamt ist damit gezeigt, dass die klassische Aviditätsmessung bei SARS-CoV-2 zu keiner sinnvollen und sicheren Differenzierung zwischen akuter und zurückliegender Infektion in der Lage ist.*
- *Die regelmäßige Koppelung der IgG-Befunde gegen die drei verwendeten viralen Antigene stellt einen bemerkenswerten Befund dar.*

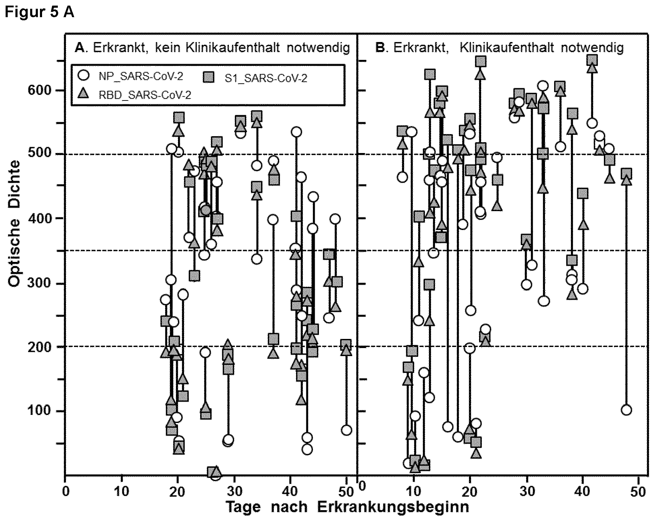

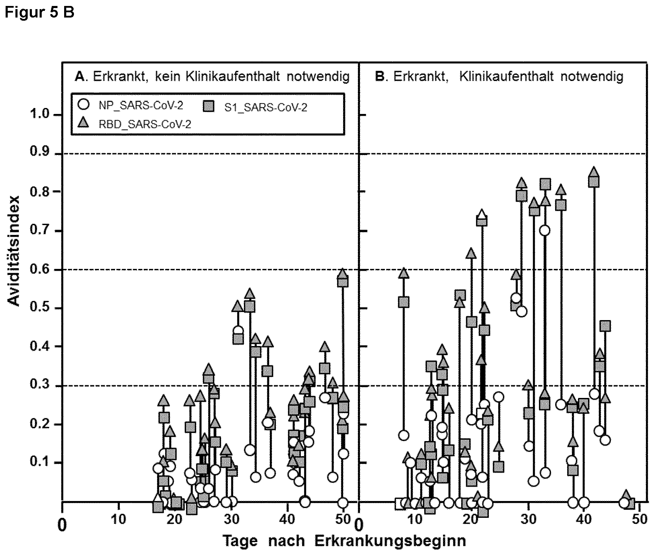

Ein erster Vergleich der serologischen Befunde von COVID-19-Patienten, deren Krankheitsverlauf keinen Klinikaufenthalt notwendig machte, mit Patienten, die in der Klinik behandelt werden mussten (zum Teil auf Intensivstation), zeigt, dass bei einigen der Klinik-pflichtigen Patienten die Werte der optischen Dichte der Antikörperbestimmung höher lagen als bei der Vergleichsgruppe (Figur 5A). Der Anstieg wird besonders im Segment der optischen Dichte > 500 deutlich und wird von einem damit korrespondierenden Verlust im Segment < 200 OD-Einheiten begleitet (Figur 5 A). Der Anstieg der OD korreliert mit einer Zunahme der Aviditätsindizes bei den Klinik-pflichtigen Patienten, der aus der Zunahme der Proben mit einem Aviditätsindex > 0.6 besonders deutlich erkennbar wird (Figur 5 B). Es fällt dabei auf, dass dabei Werte mit hoher Avidität besonders für Antikörper gegen RBD und S1 zu finden sind.

Diese Befunde erscheinen zunächst als paradox, vor allem im Vergleich mit anderen Virusinfektionen. Sie weisen jedoch möglicherweise darauf hin, dass eine mit SARS-CoV-2 infizierte Person, die die anfängliche lokale Vermehrung des Virus im Respirationstraktes nicht wirkungsvoll einzudämmen vermag, einerseits durch die länger anhaltende Virusvermehrung das Risiko einer schwerwiegenderen Erkrankung erleidet, andererseits durch die potenziell größere verfügbare Konzentration an Virusproteinen die Chance hat, eine starke humorale Immunantwort mit voller Aviditätsreifung zu entwickeln. Diese könnten dann an der Gesundung mitwirken und vor einer eventuellen Zweitinfektion schützen. Die Klärung dieses Sachverhalts bedarf der genauen Bestimmung des Grades der Aviditätsreifung, der mithilfe des hier vorgestellten Verfahrens erreicht werden kann. Damit ist für unser Mess-System auch eine potenzielle Möglichkeit von prospektiven Aussagemöglichkeiten erkennbar.

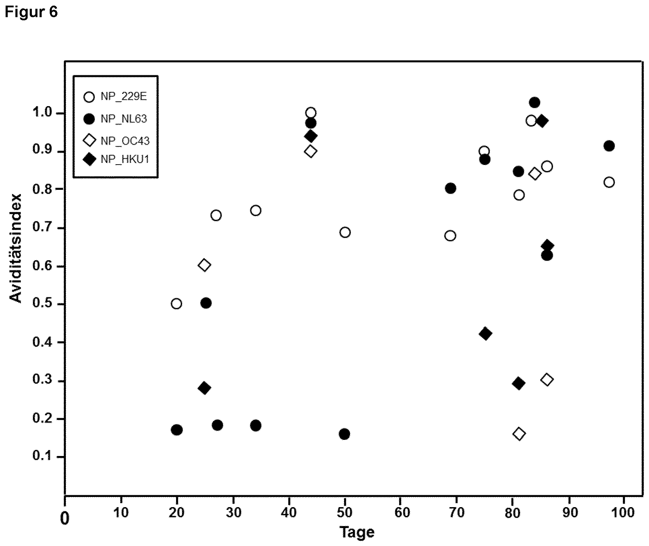

Neu ist auch der überraschende Befund, dass das Ausbleiben einer vollständigen Aviditätsreifung auch bei der Immunantwort gegen saisonale Coronaviren (229E, NL63, OC43, HKU-1) zu beobachten ist (Figur 6), wenn auch mit geringerer Ausprägung als dies für SARS-CoV-2 gesehen wird.

### Beispiel 2

### Darstellung der Dynamik der Antikörperantwort durch systematische Variation der bei der Aviditätsbestimmung verwendeten Harnstoffkonzentrationen

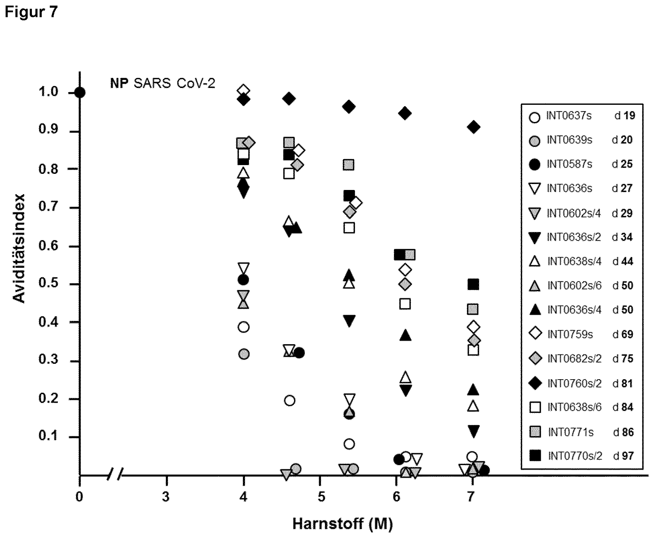

In dem in Figur 7 gezeigten weiteren Versuch wurde die Aviditätsbestimmung des IgG gegen das Nukleoprotein (NP) von SARS-CoV-2 mit einer Serie von definierten Harnstoffkonzentrationen (4 M, 4.6 M, 5.3 M, 6.1 M und 7M) mithilfe des Lineassays durchgeführt. Dabei wurden drei Gruppen von Patienten mit SARS-CoV-2-Infektion aufgrund des Entnahmedatums der Seren getrennt analysiert (A: 12-29 Tage, B: 30-69 Tage und C: mehr als 70 Tage nach Beginn der Symptome). Es zeigt sich, dass der Grad der Aviditätsreifung sehr variabel ist, aber dennoch relativ genau bestimmt werden kann. Auch wenn mit einer Ausnahme (Serum mit der ID INT0760S/2) keine klassische hohe Avidität erreicht wurde, ist dieses analytische Verfahren mit seinen veränderten Harnstoffkonzentrationen durchaus geeignet, die geordnete und begrenzte Dynamik Aviditätsreifung, wie sie nach SARS CoV-2-Infektionen typisch zu sein scheint, darzustellen.

Figur 7 zeigt die hohe Variabilität der Aviditätsreifung des IgG gegen SARS-CoV-2 Nukleoprotein, die Darstellbarkeit des Grades der Reifung und das Ausbleiben der vollständigen Reifung in der überwiegenden Mehrzahl der Fälle. Die gemessene Ausnahme (Serum mit der ID INT0760S/2) einer klassisch hohen Avidität erfüllt dabei eine besondere, wichtige Rolle: Sie zeigt, dass das virale Protein und das verwendete Testsystem tatsächlich in der Lage sind, hohe Aviditätsindizes darzustellen, wenn sie denn vorliegen. Beim Fehlen dieser Kontrolle hätte theoretisch in Betracht gezogen werden können, dass sich das Nukleoprotein von SARS CoV-2 nicht für die Aviditätsbestimmung eignen würde.

Der für SARS CoV-2 Nukleoprotein in Figur 7 erhobene Befund wiederholt sich in analoger Weise auch bei der Bestimmung der Avidität des IgG gegen RBD bzw. S1-Protein (Figuren 8 und 9). Die Befunde sind analog zu denen für IgG gegen NP, weisen jedoch graduell in Einzelfällen höhere Aviditätsindizes auf als dies für NP gefunden wurde.

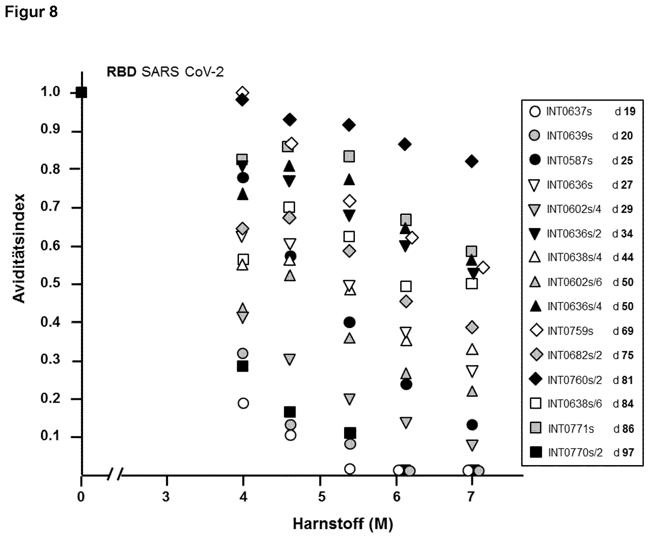

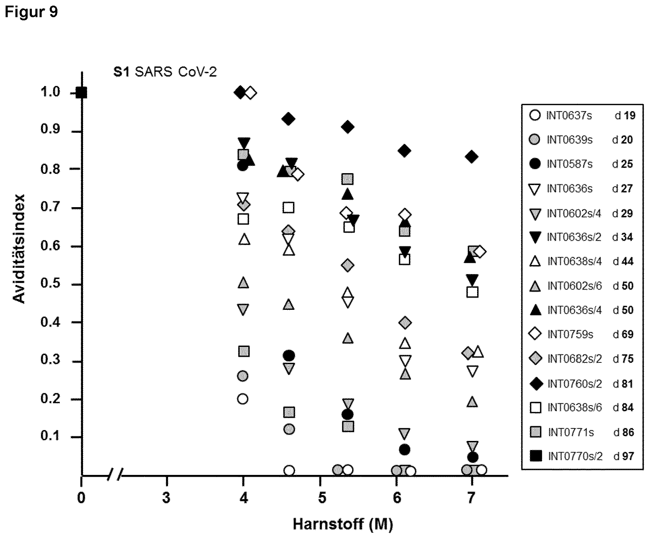

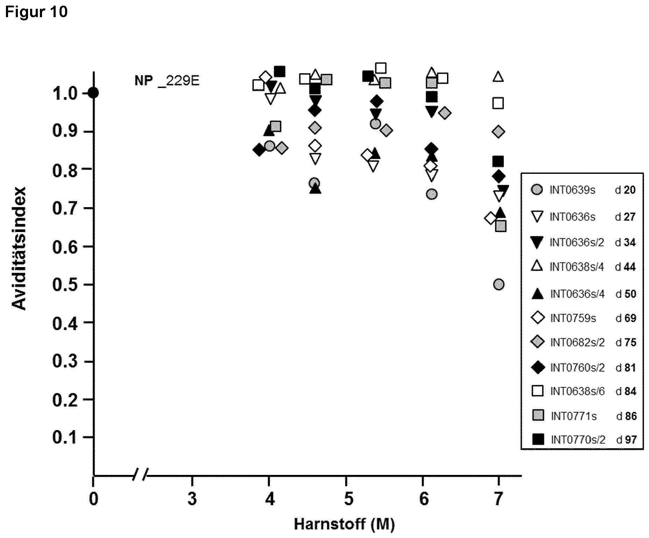

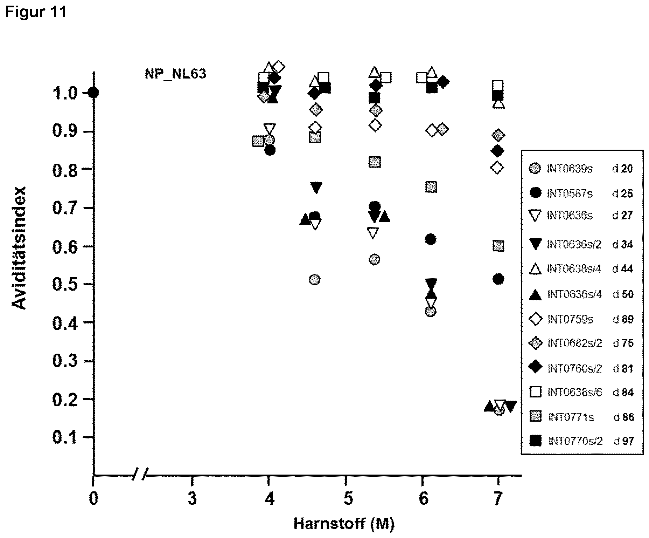

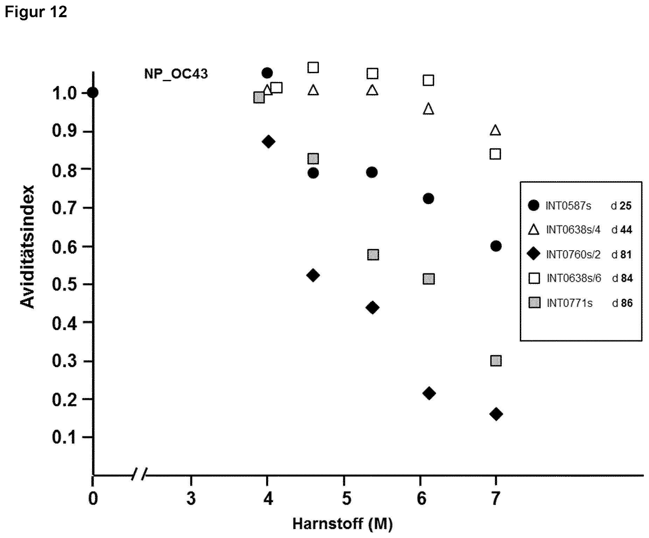

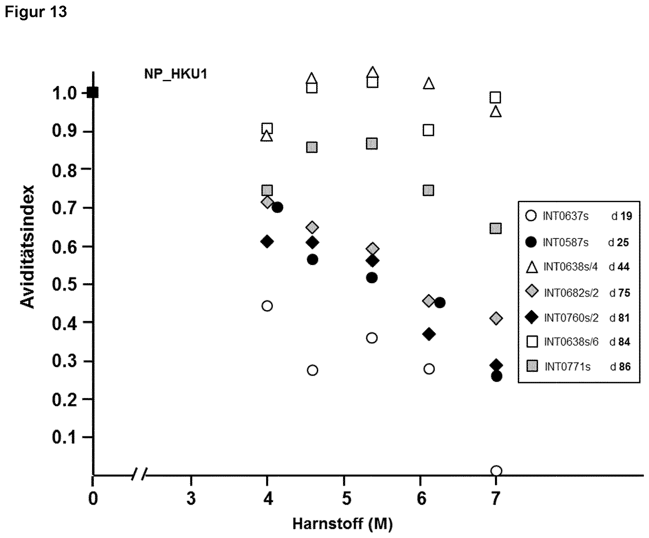

Der in den Figuren 7-9 für SARS-CoV-2 erhobene Befund lässt sich auch prinzipiell auf das NP der vier getesteten saisonalen Coronaviren 229E, NL63, OC43 und HKU-1 übertragen (Figuren 10-13), wobei sich deutliche graduelle Unterschiede zwischen den Ergebnissen für die einzelnen Virussystemen zeigen. Gemeinsamer Nenner bleibt der Befund, dass in zahlreichen Fällen von Coronavirusinfektion keine hohe Avidität des IgG gegen virale Proteine erreicht wird.

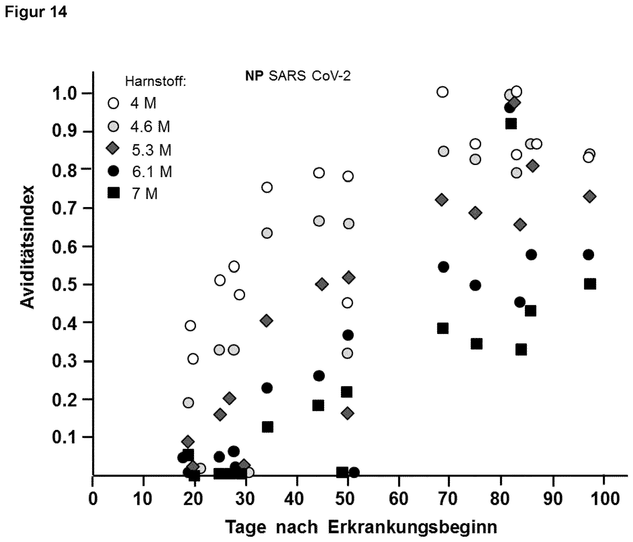

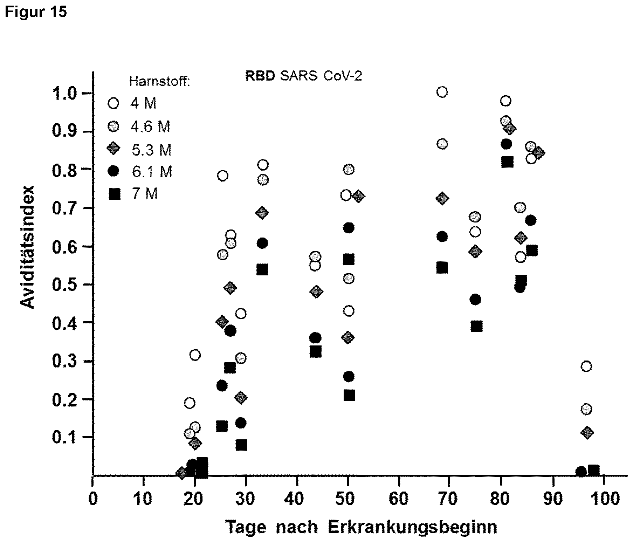

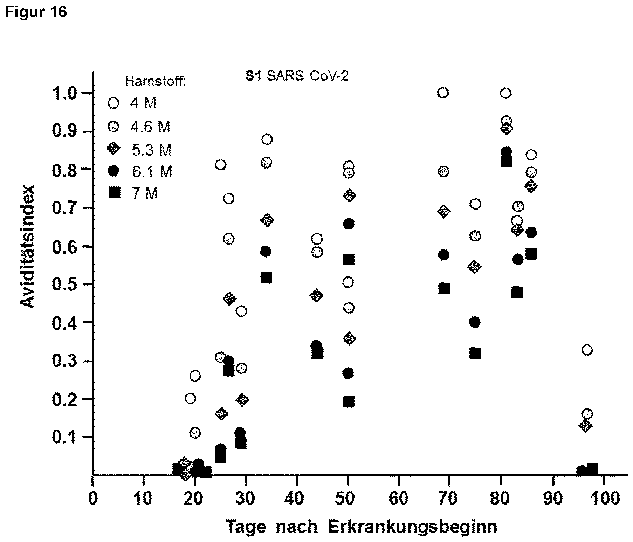

Die Darstellung der Aviditätsbefunde in der in den Figuren 14-16 gezeigten Form, bei der die Tage nach Beginn der Erkrankung und der dazu gehörende Aviditätsindex, geordnet nach den verwendeten Harnstoffkonzentrationen aufgetragen wurde, bestätigt die zugrunde liegende Dynamik. In Figur 14 wird für IgG gegen NP sogar erkennbar, dass mit einer Harnstoffkonzentration von 5.3 M eine akzeptable Trennung von akuten und länger zurückliegenden Infektionen erreichbar erscheint. Aufgrund des stärkeren Grades der Variabilität der Aviditätsreifung erscheint dies allerdings für IgG gegen RBD und S1 (Figuren 15, 16) nicht gut erreichbar. Dennoch wird aus diesen Daten die aktive Auseinandersetzung des Immunsystems mit dem SARS CoV-2 erkennbar, auch wenn in den seltensten Fällen eine für das IgG gegen andere Viren bekannte hohe Avidität nicht erreicht wird.

### Beispiel 3

### Dynamik und Abbruch der Aviditätsreifung von IgG gegen Coronaviren, insbesondere SARS CoV-2.

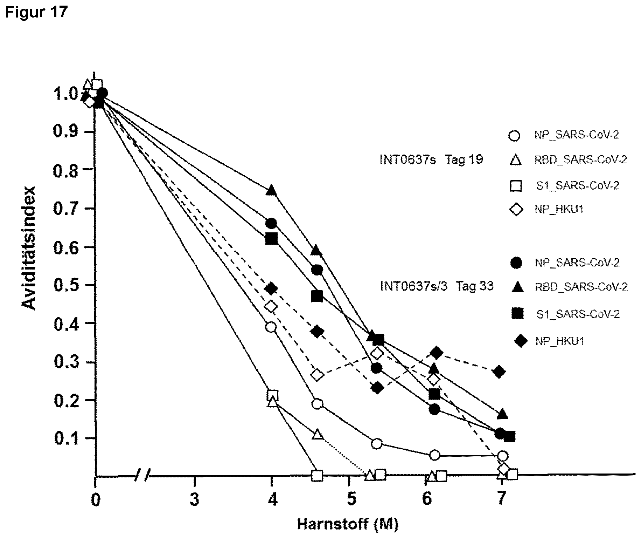

Das in Figur 17 vorgestellte Fallbeispiel belegt, dass es innerhalb von zwei Wochen (zwischen Tag 19 und Tag 33 nach Erkrankungsbeginn) zu einer sehr klar zu bestimmenden, markanten Verschiebung der Aviditätsindizes kam, und dass dieser Sachverhalt durch die Variation der Harnstoffkonzentrationen bestimmt werden kann. Bei diesem Fall liegen die Werte für die drei verwendeten viralen Antigene (NP, RBD und S1) jeweils sehr nahe beieinander.

Dieses Beispiel belegt a) die gute Messbarkeit der Avidität mit unserem Messsystem und b) die geordnete und gekoppelte Dynamik des Reifungsprozesses der Avidität des IgG gegen jedes einzelne der viralen Antigene.
Diese Analytik mit ihren zahlreichen verschiedenen Harnstoffkonzentrationen ist für die Etablierung der Messverfahren hervorragend geeignet, nicht aber für den Einsatz in der Routinediagnostik für große Fallzahlen.

Allerdings lassen die Befunde den Schluss zu, dass bei Verwendung einer Harnstoffkonzentration von 5.3 (oder geringfügig darunter oder darüber) sehr wohl die Dynamik der Reifung darstellbar ist, auch wenn insgesamt keine hohe Avidität erreicht wird. Der Bestimmung dieser Dynamik, als Ausdruck der Auseinandersetzung des Immunsystems mit dem SARS-CoV-2 stellt jedoch einen Wert an sich für die Diagnostik dar, auch wenn diese Art der Bestimmung vom klassischen Anwendungsschema der Aviditätsbestimmung aus den genannten Gründen abweicht.

Das Serum des in Figur 16 vorgestellten Patienten enthielt auch IgG gegen das saisonale Coronavirus HKU1. Wie in Figur 17 belegt ist, zeigt sich für dieses IgG trotz niedriger Avidität keine Dynamik innerhalb der Tage 19 bis 33 der Erkrankung mit COVID-19.

Dieser wichtige Befund zeigt, dass es auch bei der Immunantwort gegen saisonale Coronaviren zu einem Abbruch der Aviditätsreifung zu kommen scheint. Es darf angenommen werden, dass die Infektion mit HKU1 vor der Infektion mit SARS-CoV-2 stattfand und das Ende der Aviditätsreifung schon zum Zeitpunkt der SARS CoV-2 Infektion erreicht worden war.

Die Aufklärung derartiger komplexer Ereignisse können vorhersagbar mit der hier vorgestellten Vorgehensweise in Angriff genommen werden.

### Beispiel 4

### Nachweis des Abbruchs der Aviditätsreifung nach SARS CoV-2 Infektion

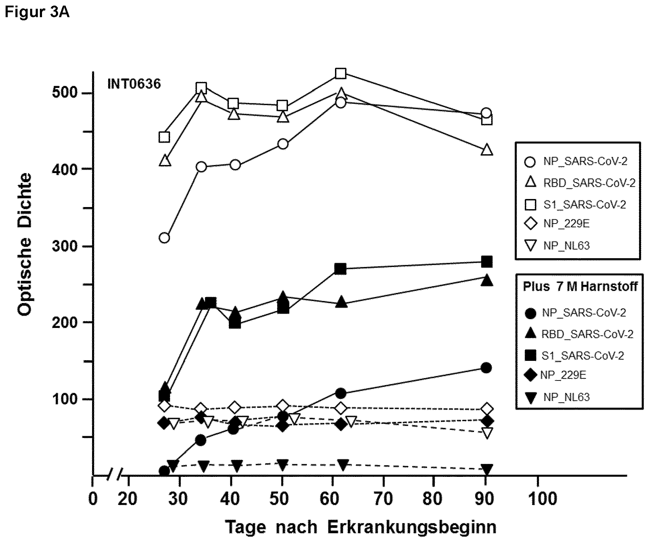

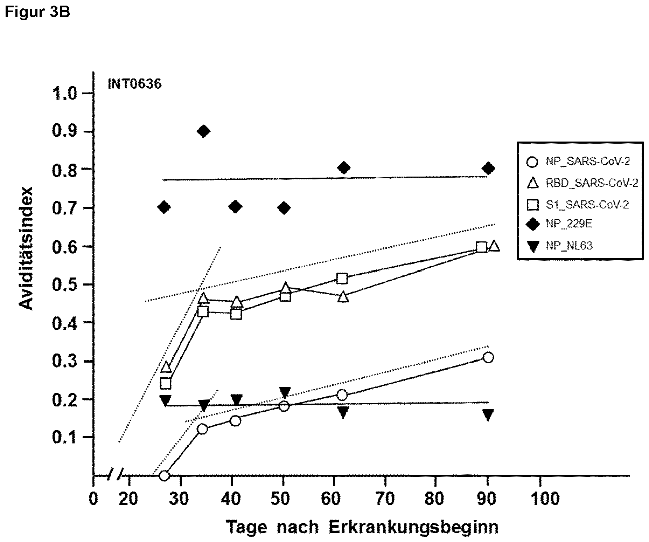

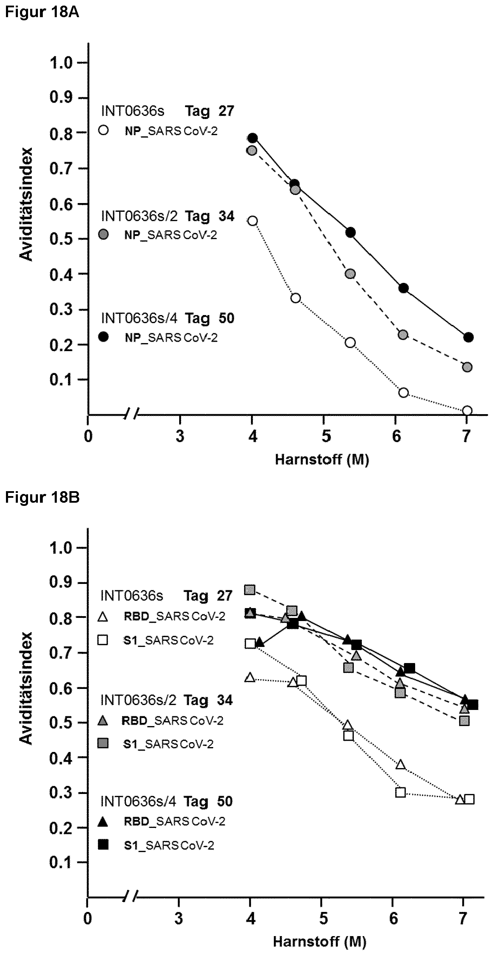

Die Analyse der Aviditätsreifung des IgG nach einer SARS CoV-2-Infektion 27 Tage, 34 Tage und 50 Tage nach Erkrankungsbeginn des Patienten zeigt, dass innerhalb der ersten Woche nach dem ersten Messpunkt eine partielle Aviditätsreifung für IgG gegen jedes einzelne Virusprotein erreicht wurde (Figur 18 A, B). Danach brach die Aviditätsreifung offensichtlich ab, denn der Vergleich der Werte für Tag 34 und Tag 50 zeigt kaum noch eine nennenswerte Verschiebung. Dieser Abbruch der Aviditätsreifung korreliert eindrucksvoll mit dem in Figur 3 für denselben Fall gezeigten Übergang der IgG-Produktion in den Stillstand.

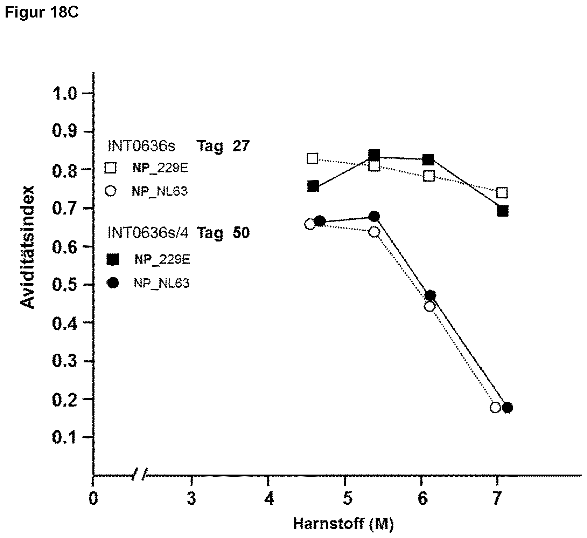

Figur 18 C zeigt sehr eindrucksvoll, dass das IgG gegen das NP der saisonalen Coronaviren 229F und NL63 im gesamten Beobachtungszeitraum zwischen 27 und 50 Tagen keine Dynamik der Aviditätsreifung erkennen ließ.

Dies zeigt, dass die Aviditätsreifung für diese IgG-Populationen schon abgeschlossen gewesen sein muss, als sich die Infektion mit SARS CoV-2 ereignete. Insbesondere die niedrige Avidität für IgG gegen NP von NL63 belegt, dass auch die Aviditätsreifung nach Infektion mit diesem saisonalen Corona-Virus im unvollständigen Status beendet worden war.

Diese Daten belegen die hohe Variabilität der Aviditätsreifung von IgG gegen Coronaviren, insbesondere SARS CoV-2, und die hohe Wahrscheinlichkeit des untypischen Abbruchs der Aviditätsreifung bei diesen Viren. Dieser Sachverhalt ist durch die Analyse zahlreicher zusätzlicher Fälle, die hier nicht im Einzelnen vorgestellt werden weiter erhärtet worden. Die hohe Variabilität der serologischen Antwort nach SARS CoV-2 Infektionen sowie das Ausbleiben der Aviditätsreifung wurden dabei weiter bestätigt. Weiterhin zeigen die Befunde, dass bei der Immunantwort gegen saisonale Coronaviren eine ausgereifte Avidität häufiger erreichbar scheint als für SARS CoV-2, dass aber auch bei diesen Viren die Aviditätsreifung in einer nicht geringen Zahl von Fällen vorzeitig beendet wird.

### Beispiel 5

### Das Problem kreuzreagierender Antikörper bei der SARS CoV-2-Diagnostik: Lösungsmöglichkeiten durch Harnstoffbehandlung

Aufgrund der häufigen Infektionen mit saisonalen Coronaviren und der Verwandtschaftsbeziehung dieser Viren mit SARS CoV-2 war es nicht unerwartet, dass in Testverfahren für IgG gegen SARS CoV-2 Spezifitätsprobleme aufgrund von Antikörpern gegen saisonale Coronaviren auftreten könnten. Deren mögliche Kreuzreaktion mit SARS CoV-2-Antigenen wurde daher als ernstzunehmendes diagnostisches Problem früh erkannt und diskutiert.

Die gängige Vorstellung zur Natur kreuzreagierender Antikörper beruht auf der Bindung einer IgG-Population, die zum Beispiel gegen ein bestimmtes Epitop auf dem Antigen eines saisonalen Coronavirus gerichtet ist mit einem verwandten Epitop eines homologen Proteins bei SARS CoV-2. Bei Annahme einer Homologie und unter der gängigen Annahme der fehlenden absoluten Identität der Epitope und ihrer unterschiedlichen Umgebung auf den jeweiligen Proteinen konnte angenommen werden, dass die Bindungsstärke eines kreuzreagierenden Antikörpers mit einem heterologen Epitop entsprechend niedrig sein sollte. Aus dieser Annahme ließ sich ableiten, dass kreuzreagierende Antikörper möglicherweise leichter mit Harnstoff vom Antigen abwaschbar sein könnten als genuine Antikörper, selbst wenn diese noch niedrig avide wären.

Als Arbeitshypothese für diese denkbare Differenzierungsmöglichkeit wurde in Erwägung gezogen, diese Differenzierung durch extrem niedrige Harnstoffkonzentrationen zu bewerkstelligen.

Zur Absicherung dieser Arbeitshypothese wurden zunächst mehrere Seren von genuinen SARS-CoV-2-Infektionen in einen Ansatz gebracht, in dem eine serielle Verdünnung von Harnstoff mit 2.5 M beginnend und aufsteigend auf das IgG aus dem Serum eines Patienten mit gesicherter SARS CoV-2-Infektion angewandt wurde. Diese Kontrolle sollte die grundsätzliche Durchführbarkeit des geplanten Ansatzes klären. Sie sollte prüfen, ob genuine Antikörper von sehr niedrigen Harnstoffkonzentrationen nur in einem unwesentlichen Umfang vom Antigen abgelöst werden. Damit erschiene der beabsichtigte Ansatz tatsächlich machbar.

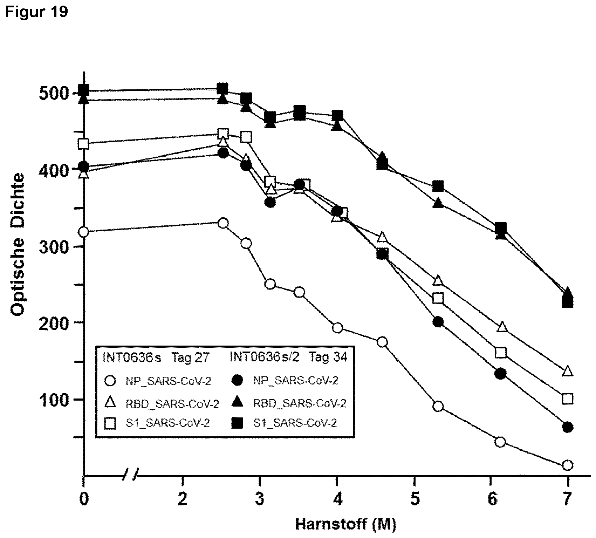

Wie die Figur 19 zeigt, führten Harnstoffkonzentrationen bis in den Bereich um 3 M tatsächlich zu keiner nennenswerten Absenkung der Bindung der spezifischen Antikörper.

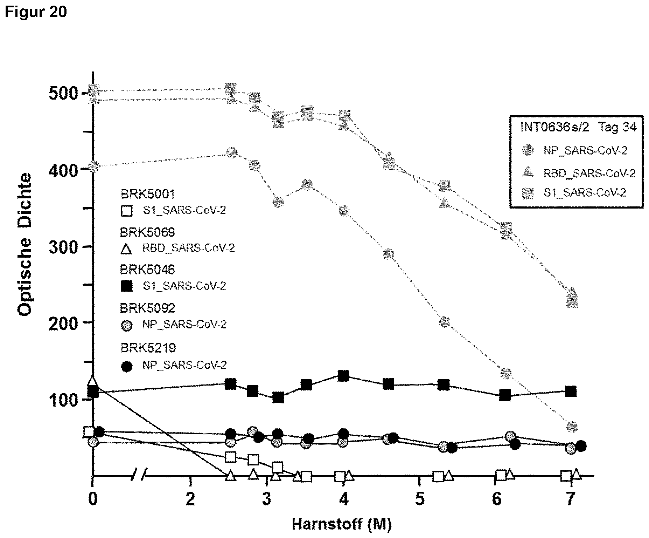

Nun konnte der Test mit fünf kreuzreagierenden Seren durchgeführt werden, die aus einer Kontrollgruppe von 450 Blutspendern mit Serumgewinnung vor der SARS CoV-2-Pandemie aufgrund eines positiven Signals für SARS CoV-2 herausgefiltert worden waren. Das Ergebnis ist in Figur 20 zusammengefasst:
Zunächst fällt auf, dass alle fünf "kreuzreagierenden" Seren zu positiven Befunden nur knapp über dem Cut-off geführt hatten. Die Konzentration kreuzreagierender Antikörper im Serum scheint daher nur gering zu sein. Weiterhin fällt auf, dass diese Seren ausnahmslos nur gegen ein einziges virales Antigen gerichtet waren. Dies konnte NP, RBD oder S1 sein. Im Gegensatz zu diesem bemerkenswerten Befund war bei jedem der vorher untersuchten echten SARS CoV-2-Infektionen stets eine Immunantwort gegen alle drei eingesetzten Antigene gefunden worden (Figur 4). Diese strikte Koppelung der drei Marker betraf auch die Fälle, in denen die Antikörperkonzentration relativ gering war.

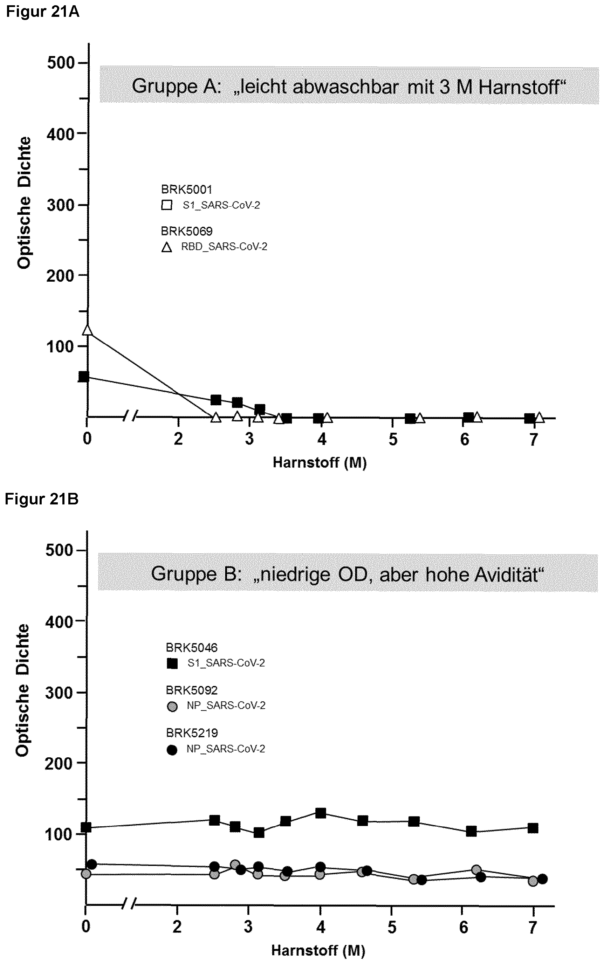

Die in Figur 20 vorgestellten kreuzreaktiven Seren konnten in zwei klar zu trennende Gruppen zusammengefasst werden. Bei zwei Seren konnten die Antikörper gegen SARS-CoV-2 Antigene mit 3 M Harnstoff fast vollständig abgewaschen werden (Figur 21 A), was die Einstufung der Reaktion dieser Seren als klassische Kreuzreaktionen zulässt. Auf jeden Fall können diese Seren im Vergleich mit den Befunden echter SARS CoV-2-Infektionen nicht als sichere SARS CoV-2-Infektionen gelten.

In Hinblick auf die medizinische und epidemiologische Problematik von COVID-19 sollte jedoch bei ähnlichen zukünftigen Befunden unter Bedingungen unter denen eine SARS CoV-2 Infektion theoretisch möglich wäre eine alternative theoretische Annahme ebenfalls ernstgenommen werden, auch wenn sie relativ unwahrscheinlich erscheint: Es könnte sich bei diesen Seren um Fälle handeln, bei denen eine extrem frühe Infektion gerade erkennbar ist und daher sowohl bezüglich der Antikörperkonzentration und deren Avidität äußerst niedrig wäre. Gleichzeitig wären aus kinetischen Gründen nur messbare Antikörper gegen ein einziges Antigen nachweisbar. Durch eine serologische Kontrolluntersuchung im Abstand von 2-3 Wochen, mit parallelem Versuch des Erregernachweises durch PCR und adäquate Quarantäne in diesem Zeitraum, könnte diesen seltenen Fällen korrekt Rechnung getragen und ihr wahrer Serostatus sicher bestimmt werden.

Die zweite Gruppe von kreuzreagierenden Antikörpern zeigte ein bemerkenswertes und unerwartetes Profil (Figur 21 B): Auch hier ist die Antikörperkonzentration sehr gering, was sich in einem Wert der optischen Dichte knapp über dem Cut-Off-Wert ausdrückt. Und auch bei diesen Seren ist die Immunantwort nur gegen ein einziges SARS-CoV-2 Protein gerichtet. Bemerkenswert ist nun, dass bei diesen drei Seren eine sehr hohe Avidität vorliegt. Damit ist ihre Differenzierung von gerade begonnen SARS-CoV-2 Infektionen sehr klar und einfach zu erreichen. Aufgrund der Herkunft der Seren ist dies ohnehin schlüssig und ein Hinweis darauf, dass es sich um hochavide Antikörper gegen bestimmte Epitope definierter Proteine von saisonalen Coronaviren handeln muss. Die Tatsache, dass diese Antikörper auch im SARS CoV-2-Test als hochavid erkannt werden, lässt sich nur unter der Annahme erklären, dass es sich wohl um seltene Epitope handeln muss, die in identischer Form bei saisonalen Coronaviren und bei SARS CoV-2 vorhanden sind. Aufgrund der hier gezeigten Charakteristika lassen sich diese Signale jedoch klar von einer spezifischen Immunantwort auf SARS-CoV-2 abgrenzen. Der Begriff "spezifisch" ist hier in dem Sinne zu verstehen, dass die Immunantwort von Antigenen des gleichen Virus ausgelöst wurde gegen das auch getestet wird. Die "Passgenauigkeit" von kreuzreagierenden Antikörpern in Bezug auf das homologe Antigen des verwandten Virus wird dabei nicht in Frage gestellt, d. h. die Bindungsreaktion als solche ist durchaus als spezifisch zu werten. An dieser Stelle wird die Grenze einer präzisen sprachlichen Differenzierung fast erreicht.

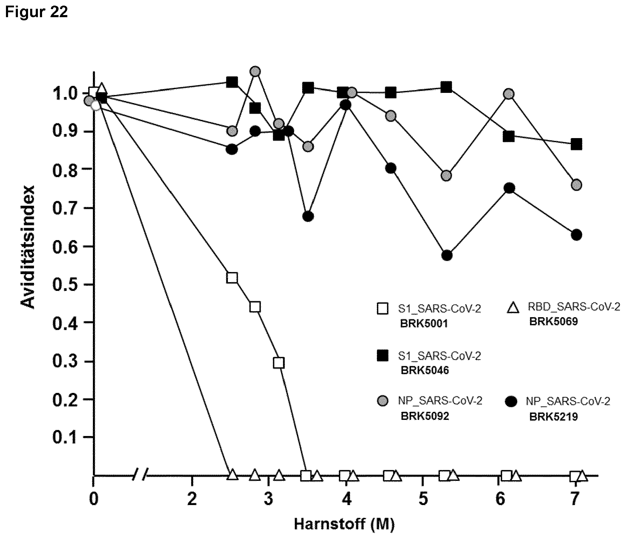

Die Betrachtung des jeweiligen Aviditätsindex der kreuzreagierenden Antikörper (Figur 22) verdeutlicht die Klarheit, die der Differenzierung der beiden Gruppen dieser Antikörper zugrunde liegt.

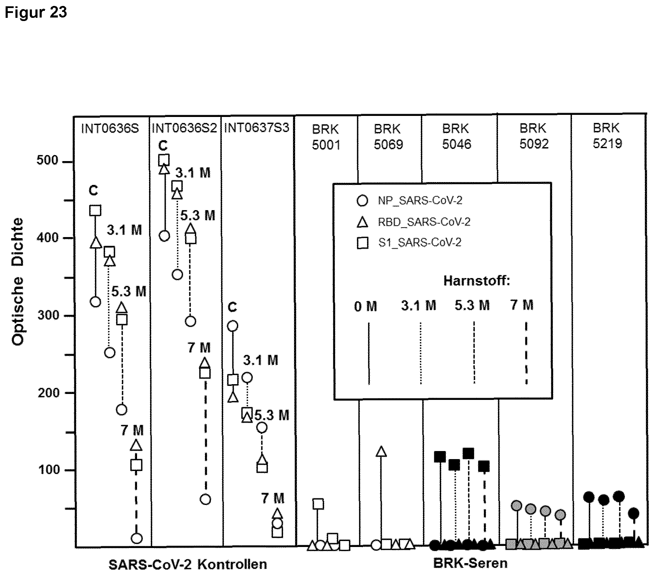

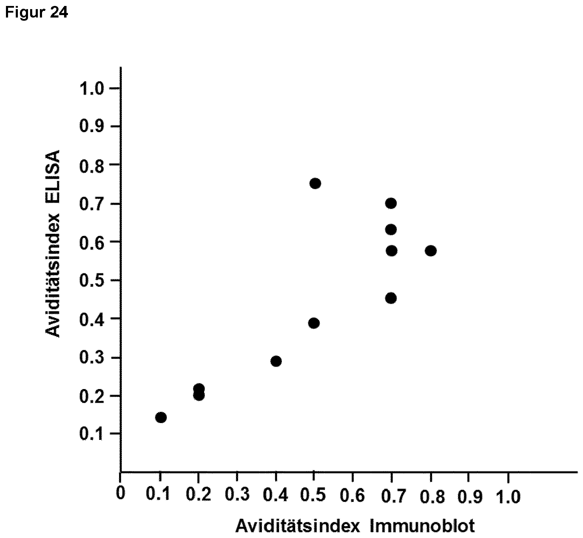

Figur 23 stellt die Charakteristika genuiner Immunantworten gegen SARS-CoV-2 denen der beiden Gruppen von kreuzreagierenden Antikörpern, die durch saisonale Coronaviren induziert wurden, gegenüber.

Bemerkenswert sind die unterschiedlichen erreichten Konzentrationen, die Besonderheiten nach Harnstoffbehandlung sowie der gravierende Unterschied bezüglich der Koppelung der Immunantwort gegen verschiedene Antigene. Es gilt bei der Diskussion zu beachten, dass die Koppelung der Antwort gegen alle drei getesteten Antigene bei den echten SARS-CoV-2-Infektionen als sehr strikt gefunden wurde (Figur 4).
- *Durch die rationale Anwendung dieser Kriterien lässt sich vorhersagbar eine deutliche Steigerung der Spezifität des Antikörpertests erreichen. Diese ist in Hinblick auf die relative Seltenheit der SARS CoV-2-lnfektion in vielen Gebieten essentiell für adäquate Vorsorgemaßnahmen aufgrund einer präzis ermittelten Inzidenz für Infektionen.*
- *Damit kann das hier vorgestellte Testsystem trotz der unerwarteten Besonderheiten der SARS-CoV-2-Serologie nicht nur aktive SARS CoV-2 Infektionen sichern, sondern sollte auch zu einer deutlichen Steigerung der Spezifität der Bestimmung der Infektion mit SARS CoV-2 beitragen.*

### Beispiel 6

### Prospektive Rolle der Aviditätsbestimmung für die Überprüfung der Immunität gegenüber SARS CoV-2.

Die hier präsentierten Daten zeigen eine Korrelation zwischen der besonderen Kinetik der Antikörperantwort nach SARS CoV-2-Infektion, die meist nach einer Plateauphase einen Rückgang der Antikörperkonzentration zeigt, mit ihrem auffällig häufigen Abbruch der Aviditätsreifung der Antikörper. Als denkbares Resultat dieser Situation ist zu diskutieren, ob sich aus der Verringerung der Antikörperkonzentration nicht auch ein Verlust der Immunität und damit die Gefahr einer Reinfektion ergeben könnte.

Hinter diesem Szenario könnte sich eine besondere Strategie von SARS CoV-2 und Coronaviren im Allgemeinen verbergen, die darauf abzielt, durch Minimierung der für das Immunsystem verfügbaren Antigenmenge die Antikörperproduktion und die Affinitätsreifung zu drosseln. Mechanistisch ist ein solcher Mechanismus durch das Vermeiden einer starken Virämie, lokal begrenzte Infektionsorte und die daraus resultierende abortive humorale Immunantwort im Prinzip gut erklärbar. Dieses Szenario würde es den Coronaviren ermöglichen, in zeitlichen Abständen neue Zyklen von Neuinfektionen zu erreichen und damit das Verbleiben der Viren in der Wirtspopulation auf lange Sicht zu sichern.

Bisher wird vor allem die Antikörperkonzentration (insbesondere die der neutralisierenden Ak) als wichtiges Modul der Regulation der Immunität diskutiert. Die Literatur beinhaltet jedoch viele etablierte Beispiele, die belegen, dass der Avidität der Antikörper ("funktionelle Affinität") bei diesen protektiven Vorgängen die eigentliche zentrale Bedeutung zukommt. In mehreren Virussystemen wurde gezeigt, dass das Ausbleiben einer vollständigen Affinitätsreifung mit der Gefahr eine spätere Infektion nicht verhindert werden kann - trotz der Anwesenheit von niedrigaviden Antikörpern in deutlich messbarer Konzentration.

Für SARS CoV-2 wurde inzwischen gezeigt, dass es zu einem Antikörperverlust und dem Verlust der Immunität trotz erfolgter Infektion kommen kann. Dabei war der Verlust bei milden oder klinisch inapparenten Verläufen früher zu beobachten als nach erfolgreich überstandenen schweren Verläufen. Diese Befunde decken sich mit den vorliegenden Befunden zur Kinetik der Antikörperantwort gegen SARS CoV-2.

Bedeutet dies nun, dass die für die Eindämmung der Pandemie notwendige Immunisierung gegen SARS CoV-2 nur ein Wunschtraum ist und an der biologischen Wirklichkeit scheitern muss? Gilt nicht die Grundregel dass eine durchgemachte Infektion mit einem Virus der Immunisierung qualitativ überlegen ist?

Aus dem Mechanismus der Aviditätsreifung lässt sich vorhersagen, dass die Immunisierung gegen SARS CoV-2 durchaus eine höhere protektive Qualität erzielen könnte als die Infektion mit dem Virus. Es ist vorhersagbar und rational zu begründen, dass eine Impfstrategie, die zu einem langanhaltenden, optimalen Antigenspiegel führen würde, die Aviditätsreifung bis zur Aviditätsreife antreiben könnte. Dieser Prozess könnte zum gewünschten Impferfolg, nämlich ausreichendem Schutz durch Immunität, führen.

Wie könnte ein derartiger, wünschenswerter Impferfolg überprüft werden? Es wird angenommen, dass die Protektion mit der Avidität der protektiven Antikörper korreliert. Daher ist die Bestimmung der Avidität der Antikörper für die Bedeutung der Immunität höchstrelevant.

Das hier vorgestellte Testsystem besitzt alle notwendigen Eigenschaften, um in der Lage zu sein, hochavides, funktionell protektives IgG nach Immunisierung von schlechter wirksamen niedrigaviden IgG zu unterscheiden. Vorbehaltlich einer Überprüfung von Seren immunisierter Personen, sobald diese verfügbar sind, darf bereits jetzt angenommen werden, dass der Einsatz des Aviditätstests mit etwa 6 M - 7 M Harnstoff eine Antwort auf die qualitativ hochwertige Protektion geben sollte.

Das Testsystem sollte auch in der Lage sein, eine protektive Immunantwort durch genuine Entwicklung von IgG gegen SARS CoV-2 von einer anamnestischen Reaktion von Gedächtniszellen für IgG gegen entsprechende Antigene von saisonalen Coronaviren zu unterscheiden. Es ist nämlich denkbar, dass bei der Immunisierung mit SARS CoV-2 Antigenen immunologische Gedächtniszellen erreicht und zur Proliferation angeregt werden, die hochavide Antikörper gegen das Oberflächenprotein von *saisonalen Coronaviren* generieren können (und die im Zuge einer Infektion mit diesen saisonalen Coronaviren gebildet wurden). Diese Gedächtniszellen können nur dann von SARS-CoV-2-Antigenen stimuliert werden, wenn hochaffine Kreuzreaktivität besteht. Es ist sehr wahrscheinlich, dass die in diesem Szenario gebildeten Antikörper auch tatsächlich "kreuzprotektiv" gegen SARS CoV-2 wirken. Es ist weiterhin zu bedenken, dass bei Vorliegen limitierender Konzentrationen an Antigen die kreuzreagierenden Gedächtniszellen das Antigen effizienter an sich binden als die B-Zellen der durch die Immunisierung stimulierten "genuinen" B-Zellen mit IgG gegen SARS-CoV. Deren zunächst niedrigavide Antikörperantwort könnte in einem nicht vorhersagbaren Grad gehemmt oder sogar unterbunden werden, zu Gunsten der von Anfang an hochaviden kreuzprotektiven Antikörper. Die Konsequenz für die Protektion der geimpften Person kann verschieden diskutiert werden. Es steht jedoch fest, dass der erfindungsgemäße Testansatz auch im Einzelfall den Sachverhalt der Impfsituation unter Berücksichtigung dieser komplexen Interaktion klären können sollte.

## Patentansprüche

1. Verfahren zur Bestimmung der Avidität von gegen ein Coronavirus gerichteten Antikörpern, das folgende Schritte umfasst:
a) Bereitstellung von mindestens zwei festen Phasen, wobei auf jeder festen Phase in gleicher Weise und in gleicher Menge wenigstens zwei verschiedene Antigene von SARS - CoV-2 immobilisiert sind, und auf jeder festen Phase die verschiedenen Antigene räumlich voneinander getrennt sind,
b) Inkubation der festen Phasen aus a) mit einer Antikörper enthaltenden Lösung,
c) Inkubation einer der festen Phasen in einer Lösung, die nicht die Bindung von Antikörpern mit niedriger Avidität an die Antigene wesentlich reduziert, sowie
d) Inkubation mindestens einer weiteren festen Phase in mindestens einer Lösung, wobei diese Lösung Harnstoff in einer Konzentration aufweist, die die Bindung von Antikörpern mit niedriger Avidität an die Antigene reduziert, und
e) Detektion der an die festen Phasen gebundenen Antikörper,
**dadurch gekennzeichnet, dass** die Konzentration an Harnstoff so eingestellt ist, dass nur ein Teil der gebundenen Antikörper abgelöst wird.

2. Verfahren nach Anspruch 1, **dadurch gekennzeichnet, dass** in Verfahrensschritt d) wenigstens zwei feste Phasen mit verschiedenen Lösungen enthalten unterschiedliche Konzentrationen an Harnstoff inkubiert werden.

3. Verfahren nach Anspruch 1 oder 2, **dadurch gekennzeichnet, dass** die in Schritt d) eingesetzten Lösungen verschiedene Konzentrationen an Harnstoff aufweisen, die zwischen 3 und 7 molar liegen.

4. Verfahren nach Anspruch 1,2 oder 3, **dadurch gekennzeichnet, dass** die Harnstoff enthaltende Lösung nach Verfahrensschritt d) und vor Verfahrensschritt e) abgewaschen wird.

5. Verfahren nach einem der Ansprüche 1 bis 4, **dadurch gekennzeichnet, dass** Harnstoff in einer Konzentration von 3,1 bis 6,9 M verwendet wird.

6. Verfahren nach einem der Ansprüche 1 bis 5, **dadurch gekennzeichnet, dass** die festen Phasen Membranen sind, die aus
einem Material bestehen, ausgewählt aus einer Gruppe enthaltend Nitrocellulose, Nylon und Polyvinylidenfluorid.

7. Verfahren nach einem der Ansprüche 1 bis 6, **dadurch gekennzeichnet, dass** die Antigene an unterschiedlichen Stellen auf die Membranen aufgetragen werden.

8. Verfahren nach einem der Ansprüche 1 bis 7, **dadurch gekennzeichnet, dass** die Detektion der Antikörper in Schritt e) durch Inkubation mit enzymgekoppelten sekundären Antikörpern und anschließende enzymatische Nachweisreaktion erfolgt.

9. Verfahren nach einem der Ansprüche 1 bis 5, **dadurch gekennzeichnet, dass** es sich um ein ELISA Testverfahren handelt, bei dem die Antigene an Mikrotiterplatten fixiert sind.

10. Verfahren nach einem der Ansprüche 1 bis 5, **dadurch gekennzeichnet, dass** es sich um ein ELISA Testverfahren handelt, bei dem die Antigene auf Kunststoff Kügelchen fixiert

11. Verfahren nach einem der vorhergehenden Ansprüche, **dadurch gekennzeichnet, dass** die auf den festen Phasen immobilisierten Antigene rekombinant hergestellt werden und ausgewählt werden aus einem SARS-Cov-2 Nukleoprotein, einer SARS-Cov-2 Rezeptor bindenden Domäne des S1 Proteins und einem SARS-CoV-2 Oberflächenprotein 1.

12. Verfahren nach einem der vorhergehenden Ansprüche, **dadurch gekennzeichnet, dass** an der festen Phase, auch Nukleoproteine von saisonalen Coronaviren, ausgewählt aus 229E, NL63, OC43 und HKU1 gebunden sind.

13. Verfahren nach einem der vorhergehenden Ansprüche, **dadurch gekennzeichnet, dass** das Ausmaß der Bindung von Antikörpern an Antigene standardisiert optometrisch bestimmt wird, so dass das Verhältnis der Bindung zwischen Antigen und Antikörper, das bei den verschiedenen Ansätzen bestimmt wird miteinander verglichen werden kann.

14. Testkit für die Durchführung eines Verfahrens nach einem der Ansprüche 1 bis 13, enthaltend mehrere Line-Assay-Streifen, auf denen Antigene von Corona-Virus immobilisiert sind, wobei auf jedem Träger in gleicher Weise und in gleicher Menge wenigstens drei verschiedene Antigene von SARS-Cov-2 und wenigstens drei Antigene von gegebenenfalls unterschiedlichen saisonalem Corona-Virus immobilisiert sind, und auf jedem Träger die verschiedenen Antigene räumlich voneinander getrennt sind **dadurch gekennzeichnet, dass** es als denaturierendes Mittel Harnstoff, der als 3,1 bis 6,9 M Lösung in Verfahrensschritt d) eingesetzt wird, enthält.

15. Verwendung rekombinant hergestellter Antigene, ausgewählt aus einem SARS-CoV2 Nucleoprotein, einer SARS-CoV2 Rezeptor bindenden Domäne des S1 Proteins, einem SARS - Cov-2 Oberflächenprotein 1 zur Bestimmung der Aviditäten von Antikörpern gegen Antigene, wobei
a) mindestens zwei feste Phasen bereitgestellt werden, auf jeder festen Phase in gleicher Weise und in gleicher Menge wenigstens zwei verschiedene Antigene immobilisiert sind, und auf jeder festen Phase die verschiedenen Antigene räumlich voneinander getrennt sind,
b) die festen Phasen aus a) mit einer Antikörper enthaltenden Lösung inkubiert werden,
c) eine der festen Phasen in einer Lösung, die nicht die Bindung von Antikörpern mit niedriger Avidität an die Antigene wesentlich reduziert, inkubiert wird,
d) mindestens eine weitere feste Phase in einer Lösung inkubiert wird, wobei diese Lösung Harnstoff in einer Konzentration aufweist, die die Bindung von Antikörpern mit niedriger Avidität an die Antigene reduziert, und
e) die an die festen Phasen gebundenen Antikörper detektiert werden,
wobei durch den Vergleich der Bindung der Antikörper an die verschiedenen Antigene die Avidität ermittelt werden kann.
